# EUROPÄISCHE PATENTANMELDUNG

(11) **EP 4 338 769 A1**
(43) Veröffentlichungstag der Anmeldung: **20.03.2024**
(21) Anmeldenummer: 23184641.1
(22) Anmeldetag: 11.07.2023
(51) Int. Cl.: A61M 5/00, B65B 69/00

(54) **VORRICHTUNG, SET UND VERFAHREN ZUM ENTNEHMEN EINES STERILEN GEGENSTANDES AUS EINEM FOLIENBEUTEL**

(30) Priorität: 16.09.2022 DE 102022123841
(71) Anmelder: Syntegon Technology GmbH, 71332 Waiblingen (DE)
(72) Erfinder: Leidig, Jürgen, 74586 Frankenhardt (DE); Ekkart, Alexander, 74564 Crailsheim (DE); Weber, Florian, 74564 Crailsheim (DE); Staeudle, Reiner, 74585 Rot am See (DE)
(74) Vertreter: DREISS Patentanwälte PartG mbB

(57) **Zusammenfassung**

Die Erfindung betrifft eine Vorrichtung (10) zum Entnehmen eines sterilen Gegenstandes (12) aus einem Folienbeutel (14) umfassend eine Nadeleinheit (16) mit einer hohlen Nadel (18) zum Einstechen in den Folienbeutel (14), einen an der Nadeleinheit (16) angeordneten Gasanschluss (20) zum Einleiten von Gas über die hohle Nadel (18) in den Folienbeutel (14), eine Halteeinrichtung (22) zum Halten des Folienbeutels (14), eine Schneideinrichtung (24) zum Aufschneiden des Folienbeutels (14), und eine Entnahmeeinrichtung (26) zum Entnehmen des Gegenstands (12) aus dem aufgeschnittenen Folienbeutel (14), wobei die Halteeinrichtung (22) mindestens eine an der Halteeinrichtung (22) flächig ausgebildete und beweglich gelagerte Halteeinheit (28) zur Kontaktierung des Folienbeutels (14) aufweist. Die Erfindung betrifft zudem ein Set (52) und ein Verfahren zum Entnehmen eines sterilen Gegenstandes (12) aus einem Folienbeutel (14).

## Beschreibung

Die Erfindung betrifft eine Vorrichtung zum Entnehmen eines sterilen Gegenstandes, insbesondere eines Tubs, aus einem Folienbeutel nach dem Oberbegriff des Anspruchs 1, ein Set mit Merkmalen des Anspruchs 11 und ein Verfahren zum Entnehmen eines sterilen Gegenstandes, insbesondere eines Tubs, aus einem Folienbeutel mit Merkmalen des Anspruchs 13.

Sterile Gegenstände werden insbesondere im medizinischen bzw. pharmazeutischen Bereich in großem Umfang verwendet. Hierbei werden bspw. sterile Einwegspritzen in einem in eine Folie verschweißten sterilen Transportbehälter, dem sogenannten Tub, gelagert und transportiert. Die sterilen Einwegspritzen müssen samt Transportbehälter bzw. Tub möglichst steril aus der Folienverpackung entnommen werden.

Die Folienverpackung ist dabei üblicherweise ein verschweißter Beutel, welcher seitlich eine überstehende Lasche aus dem Folienmaterial aufweist. Zum Öffnen eines solchen Folienbeutels wird der die Lasche aufweisende Bereich des Folienbeutels vollständig abgeschnitten oder eingeschnitten.

Durch den Unterdruck bzw. das Vakuum, das sich normalerweise im Folienbeutel vor dem Aufschneiden befindet, wird die Folie an den Transportbehälter bzw. Tub gedrückt. Dies führt nicht selten zu einem Anheften der Folie an dem Transportbehälter bzw. Tub. Damit kann es beim Entfernen des Transportbehälters bzw. Tubs aus der Folie zu Problemen mit dem Ablösen des Folienbeutels vom Transportbehälter bzw. Tub kommen.

Um eine Anhaftung des Folienbeutels zu verhindern kann dieser vor dem Aufschneiden mittels steriler Druckluft aufgeblasen werden. So kann der Folienbeutel einfacher aufgeschnitten und der Transportbehälter bzw. Tub aus dem Folienbeutel entnommen werden. Dabei wird das Anhaften des Folienbeutels am Transportbehälter bzw. Tub durch das Aufblasen des Folienbeutels möglichst verhindert.

Eine Vorrichtung, mit der ein derartiges Entnehmen eines sterilen Transportbehälters bzw. Tubs aus einem Folienbeutel möglich ist, ist aus DE 10 2011 080 289 A1 bekannt.

Nachteilig dabei ist, dass der Folienbeutel unkontrolliert aufgeblasen werden kann. Dies kann das Aufschneiden des Folienbeutels erschweren.

Es ist daher Aufgabe der vorliegenden Erfindung, eine Vorrichtung, ein Set und ein Verfahren zum Entnehmen eines sterilen Gegenstandes bereitzustellen, wobei ein kontrolliertes Aufblasen des Folienbeutels ermöglicht wird, sodass der Folienbeutel in einer definierten Form aufgeblasen wird.

Diese Aufgabe wird durch eine Vorrichtung zum Entnehmen eines sterilen Gegenstandes aus einem Folienbeutel mit den Merkmalen des Anspruchs 1 gelöst.

Bei dem Gegenstand kann es sich um einen Transportbehälter oder Tub für medizinische bzw. pharmazeutische Behältnisse, wie bspw. Fläschchen, Vials, oder (Einweg-)spritzen, handeln. Der Gegenstand kann wannenförmig ausgebildet sein.

Die Vorrichtung umfasst eine Nadeleinheit mit einer hohlen Nadel zum Einstechen in den Folienbeutel. Der Folienbeutel kann insbesondere vor dem Einstechen mit der Nadel luftdicht verschlossen bzw. verschweißt sein. Im Inneren des Folienbeutels kann ein Unterdruck bzw. ein Vakuum vorhanden sein.

Die Vorrichtung umfasst einen an der Nadeleinheit angeordneten Gasanschluss zum Einleiten von Gas über die hohle Nadel in den Folienbeutel. Bei dem Gas kann es sich um ein steriles Gas, insbesondere um sterile Druckluft, handeln. Durch das Einleiten von Gas kann der Folienbeutel aufgeblasen werden.

Die Vorrichtung umfasst eine Halteeinrichtung zum Halten des Folienbeutels. Dabei kann die Halteeinrichtung insbesondere nach dem Aufblasen des Folienbeutels mit dem Gas besonders gut an dem Folienbeutel angreifen und diesen halten.

Die Vorrichtung umfasst eine Schneideinrichtung zum Aufschneiden des Folienbeutels. Insbesondere nach dem Aufblasen des Folienbeutels mit dem Gas, kann das Aufschneiden des aufgeblasenen Folienbeutels durch einen definierten Schnitt mittels der Schneideinrichtung besonders gut umgesetzt werden.

Die Vorrichtung umfasst eine Entnahmeeinrichtung zum Entnehmen des Gegenstands aus dem aufgeschnittenen Folienbeutel. Die Entnahmeeinrichtung kann eingerichtet sein um den Gegenstand aus dem aufgeschnittenen Folienbeutel herauszuziehen. Hierzu kann die Entnahmeeinrichtung mindestens ein, insbesondere mehrere, Greif- und/oder Saugelemente aufweisen.

Ebenso denkbar ist es, dass die Entnahmeeinrichtung zum Herausschieben des Gegenstands aus dem aufgeschnittenen Folienbeutel eingerichtet sein kann.

Die Entnahme des Gegenstands mittels der Entnahmeeinrichtung kann entlang einer senkrecht zur Schwerkraftrichtung orientierten Richtung (entlang einer Waagerechten) erfolgen.

Die Halteeinrichtung weist mindestens eine an der Halteeinrichtung flächig, insbesondere eben, ausgebildete und beweglich gelagerte Halteeinheit zur Kontaktierung des Folienbeutels auf. Die Halteeinheit kann mittels mindestens eines Federelements, insbesondere Schraubenfeder, insbesondere in Schwerkraftrichtung, vorgespannt sein.

Beim Aufblasen des Folienbeutels kann dieser gegen die Halteeinheit drücken. Mittels der Halteeinheit kann der Folienbeutel beim Aufblasen in einer definierten Form gehalten werden. Dies kann sowohl das Aufschneiden des Folienbeutels als auch das Halten des Folienbeutels mittels der Halteeinrichtung erleichtern.

Gemäß einer Weiterbildung der Vorrichtung kann die Halteeinheit als Blech ausgebildet sein. Die Halteeinheit kann eine rechteckige Form aufweisen. Hierdurch kann ein möglichst einfacher und stabiler Aufbau der Halteeinheit umgesetzt werden.

Gemäß einer Weiterbildung der Vorrichtung kann die Halteeinheit senkrecht zur Schwerkraftrichtung orientiert sein. Die Halteeinheit kann insbesondere zur Kontaktierung eines in Bezug auf die Schwerkraftrichtung oberen Bereichs des Folienbeutels ausgebildet sein. Die Halteeinheit kann entlang einer senkrecht zur Schwerkraftrichtung orientierten Ebene verlaufen. Damit kann die Halteeinheit den in Bezug auf die Schwerkraftrichtung oberen Bereich des Folienbeutels in eine definierte, insbesondere flache, Form bringen. Die Halteeinheit kann quasi den in Bezug auf die Schwerkraftrichtung oberen Bereich des Folienbeutels in eine flache, senkrecht zur Schwerkraftrichtung orientierte, Form drücken.

Gemäß einer Weiterbildung der Vorrichtung kann die Halteeinrichtung mindestens eine Einsticheinrichtung mit einer Einstichnadel zum Einstechen des Folienbeutels aufweisen. Mittels der Einstichnadel der Einsticheinrichtung kann der Folienbeutel insbesondere im in Bezug auf die Schwerkraftrichtung oberen Bereich des Folienbeutels eingestochen werden. Dabei kann das durch die hohle Nadel in den Folienbeutel eingeleitete Gas aus dem Folienbeutel durch die Einstichstelle der Einstichnadel entweichen bzw. mittels der Einstichstelle der Einstichnadel ausgeleitet werden. Der Folienbeutel kann somit über die Einstichstelle der Einstichnadel gezielt entlüftet werden. Hierdurch kann ein übermäßiges Aufblasen des Folienbeutels verhindert werden, sodass der Folienbeutel kontrolliert aufgeblasen werden kann.

Die Halteeinheit kann mindestens eine Öffnung zum Durchleiten der Einstichnadel durch die Halteeinheit aufweisen.

Gemäß einer Weiterbildung der Vorrichtung kann die Einstichnadel mindestens eine Außennut zum Ausleiten des durch die Nadel in den Folienbeutel eingeleiteten Gases aus dem Folienbeutel aufweisen. Die Außennut kann auf der Außenseite der Einstichnadel angeordnet sein. Die Außennut kann eine längliche Form aufweisen. Die Außennut kann sich entlang der Längsrichtung der Einstichnadel erstrecken. Die Außennut kann sich zumindest teilweise quer zur Längsrichtung der Einstichnadel erstrecken. Die Außennut kann das Ausleiten des Gases aus dem Folienbeutel erleichtern. Die Außennut kann ein (Strömungs-)Kanal für das ausgeleitete Gas aus dem Folienbeutel bilden. Es ist denkbar, dass mittels der Form und/oder der Anzahl der Außennut(en) das Ausleiten des Gases aus dem Folienbeutel gezielt gesteuert werden kann.

Gemäß einer Weiterbildung der Vorrichtung kann die Halteeinrichtung mindestens eine Saugeinheit zum Ansaugen und/oder Halten des Folienbeutels umfassen. Die Saugeinheit kann zur Kontaktierung des in Bezug auf die Schwerkraftrichtung oberen Bereichs des Folienbeutels ausgebildet sein. Die Saugeinheit kann als Saugnapf ausgebildet sein. Hierdurch können das Ansaugen bzw. das Halten des Folienbeutels mittels einfachen Mitteln umgesetzt werden.

Gemäß einer Weiterbildung der Vorrichtung kann die Nadeleinheit eine Filtereinrichtung zum Filtern des in den Folienbeutel mittels der Nadel eingeleiteten Gases umfassen. Die Filtereinrichtung kann innerhalb der Nadeleinheit und/oder der Nadel angeordnet sein. Alternativ oder zusätzlich kann die Filtereinrichtung innerhalb des Gasanschlusses angeordnet sein. Hierdurch kann verhindert werden, dass mit dem durch die Nadel in den Folienbeutel eingeleiteten Gas Verunreinigungen in den Folienbeutel eingebracht werden und so den sterilen Gegenstand kontaminieren.

Gemäß einer Weiterbildung der Vorrichtung kann die Vorrichtung eine Bewegungseinrichtung umfassen. Die Bewegungseinrichtung kann eingerichtet sein, um die Nadeleinheit und/oder die Nadel zum Einstechen der Nadel in den Folienbeutel und Zurückziehen der Nadel aus dem Folienbeutel zu bewegen. Die Bewegungseinrichtung kann ausgebildet sein, um die Nadel insbesondere entlang der Schwerkraftrichtung hin und her zu bewegen. Hierdurch kann der Einstichvorgang mittels der Nadel mit einfachen Mitteln umgesetzt werden.

Gemäß einer Weiterbildung der Vorrichtung kann die Vorrichtung eine Fixiereinrichtung zur Fixierung eines in Bezug auf die Schwerkraftrichtung seitlichen Bereichs des Folienbeutels aufweisen. Die Fixiereinrichtung kann zur Fixierung eines eine Lasche des Folienbeutels aufweisenden Bereichs des Folienbeutels eingerichtet sein. Die Fixiereinrichtung kann zur Fixierung einer Lasche des Folienbeutels eingerichtet sein. Das Fixieren eines Bereichs des Folienbeutels mittels der Fixiereinrichtung kann das Aufschneiden des Folienbeutels erleichtern. Die Fixiereinrichtung kann, insbesondere entlang der Schwerkraftrichtung und/oder senkrecht zur Schwerkraftrichtung, beweglich ausgebildet sein.

Gemäß einer Weiterbildung der Vorrichtung kann die Fixiereinrichtung mindestens zwei Klemmelemente umfassen. Die zwei Klemmelemente können ausgebildet sein, um den Folienbeutel klemmend zu fixieren. Die zwei Klemmelemente können eingerichtet sein, um den in Bezug auf die Schwerkraftrichtung seitlichen Bereich des Folienbeutels klemmend zu fixieren. Die zwei Klemmelemente können eingerichtet sein, um die Lasche des Folienbeutels klemmend zu fixieren. Hierdurch kann das Fixieren mittels einfachen Mitteln umgesetzt werden.

Vorliegend ist mit "klemmend fixieren" gemeint, dass ein Element (bspw. Folienbeutel, Bereich des Folienbeutels oder Lasche des Folienbeutels) zwischen zwei Klemmelementen platziert wird und die zwei Klemmelemente jeweils eine Kraft aus entgegengesetzten Richtungen auf das zwischen den zwei Klemmelementen angeordnete Element ausüben. Mit anderen Worten, die zwei Klemmelemente werden zusammengedrückt (aufeinander zu bewegt), wodurch das zwischen ihnen angeordnete Element fixiert wird.

Die obige Aufgabe wird weiter durch ein Set mit den Merkmalen des Anspruchs 11 gelöst.

Das Set umfasst eine Vorrichtung gemäß obiger Ausführungen und mindestens einen Gegenstand, der von einem Folienbeutel umgeben ist. Der Folienbeutel kann ebenfalls ein Bestandteil des Sets sein.

Bei dem Gegenstand kann es sich um einen Transportbehälter oder Tub gemäß obiger Ausführungen handeln. Der Gegenstand kann mindestens eine Einbuchtung aufweisen. Die Einbuchtung kann an einer den Folienbeutel kontaktierenden Wandung des Gegenstands angeordnet sein. Die Einbuchtung kann an einer insbesondere in Bezug auf die Schwerkraftrichtung unteren und/oder zur Schwerkraftrichtung senkrecht orientierten Wandung des Gegenstands angeordnet sein.

Die Einbuchtung kann von dem Folienbeutel überspannt werden. Die Vorrichtung und/oder der Gegenstand können derart ausgebildet sein, dass die Nadel zum Einstechen in den Folienbeutel zumindest teilweise in die Einbuchtung des Gegenstands eingeführt bzw. bewegt wird. Das Einstechen des Folienbeutels mittels der Nadel kann so erleichtert werden. Zudem kann verhindert werden, dass die Nadel auf den Gegenstand trifft und dabei sich und/oder den Gegenstand beschädigt.

Hinsichtlich der mit dem Set erzielbaren Vorteile wird auf die diesbezüglichen Ausführungen zur Vorrichtung und/oder zum Gegenstand verwiesen. Zur weiteren Ausgestaltung des Sets können die im Zusammenhang mit der Vorrichtung und/oder dem Gegenstand beschriebenen und/oder die nachfolgend noch erläuterten Maßnahmen dienen.

Gemäß einer Weiterbildung der Vorrichtung kann die Halteeinheit den Gegenstand an mindestens einer Seite des Gegenstands überragen. Die Halteeinheit kann den Gegenstand an allen Seiten des Gegenstands überragen. Die Halteeinheit kann insbesondere in Blickrichtung entlang der Schwerkraftrichtung den Gegenstand an mindestens einer Seite, insbesondere an allen Seiten, des Gegenstands überragen. Hierdurch kann der aufgeblasene Folienbeutel in einer für das Aufschneiden besonders bevorzugten Form gehalten werden.

Die obige Aufgabe wird weiter durch ein Verfahren zum Entnehmen eines sterilen Gegenstands aus einem Folienbeutel mit den Merkmalen des Anspruchs 13 gelöst.

Bei dem Gegenstand kann es sich um einen Transportbehälter oder Tub gemäß obiger Ausführungen handeln.

Das Verfahren umfasst die Schritte:
Bereitstellen des in den Folienbeutel angeordneten Gegenstandes.

Einstechen des Folienbeutels mittels einer hohlen Nadel.

Einleiten von Gas über die hohle Nadel in den Folienbeutel.

Halten des Folienbeutels mittels einer Halteeinrichtung, wobei die Halteeinrichtung mindestens eine an der Halteeinrichtung flächig, insbesondere eben, ausgebildete und beweglich gelagerte Halteeinheit zur Kontaktierung des Folienbeutels aufweist.

Kontaktieren des Folienbeutels mittels der Halteeinheit. Dabei kann die Halteeinheit insbesondere einen in Bezug auf die Schwerkraftrichtung oberen Bereich des Folienbeutels kontaktieren.

Nach dem Kontaktieren des Folienbeutels mittels der Halteeinheit, weiteres Einleiten von Gas über die hohle Nadel in den Folienbeutel, sodass dieser weiter aufgeblasen wird und die Halteeinheit bewegt. Dabei kann der Folienbeutel insbesondere mit seinem in Bezug auf die Schwerkraftrichtung oberen Bereich die Halteeinheit bewegen. Dabei kann der Folienbeutel die Halteeinheit insbesondere entgegen der Schwerkraftrichtung bewegen.

Gemäß einer Weiterbildung des Verfahrens kann das Verfahren den Schritt umfassen:
Während des Einleitens von Gas über die Nadel in den Folienbeutel, Einstechen des Folienbeutels zum Ausleiten des durch die Nadel in den Folienbeutel eingeleiteten Gases aus dem Folienbeutel. Dabei kann das Einstechen des Folienbeutels insbesondere im in Bezug auf die Schwerkraftrichtung oberen Bereich des Folienbeutels umgesetzt werden.

Gemäß einer Weiterbildung des Verfahrens kann das Verfahren die Schritte umfassen:
Aufschneiden des durch die hohle Nadel aufgeblasenen und mittels der Halteeinrichtung gehaltenen Folienbeutels. Dabei kann das Aufschneiden insbesondere mittels einer Schneideinrichtung umgesetzt werden.

Entnehmen des Gegenstands aus dem Folienbeutel. Dabei kann das Entnehmen des Gegenstands insbesondere mittels einer Entnahmeeinrichtung umgesetzt werden.

Insbesondere Entsorgung des leeren und aufgeschnittenen Folienbeutels.

Gemäß einer Weiterbildung des Verfahrens kann zur Durchführung des Verfahrens eine Vorrichtung gemäß obiger Ausführungen und/oder ein Set gemäß obiger Ausführungen verwendet werden.

Hinsichtlich der mit dem Verfahren erzielbaren Vorteile wird auf die diesbezüglichen Ausführungen zur Vorrichtung und/oder zum Set verwiesen. Zur weiteren Ausgestaltung des Verfahrens können die im Zusammenhang mit der Vorrichtung und/oder dem Set beschriebenen und/oder die nachfolgend noch erläuterten Maßnahmen dienen.

Weitere Merkmale, Einzelheiten und Vorteile der Erfindung ergeben sich aus dem Wortlaut der Ansprüche sowie aus der folgenden Beschreibung eines Ausführungsbeispiels anhand der Zeichnungen. Es zeigen:
- Fig. 1: eine perspektivische Ansicht einer Vorrichtung;
- Fig. 2: einen Ausschnitt einer perspektivischen Ansicht der Vorrichtung aus Figur 1 und
- Fig. 3: einen Ausschnitt einer Seitenansicht der Vorrichtung aus Figur 1.

In der nachfolgenden Beschreibung sowie in den Figuren tragen sich entsprechende Bauteile und Elemente gleiche Bezugszeichen. Der besseren Übersichtlichkeit wegen sind nicht in allen Figuren sämtliche Bezugszeichen wiedergegeben.

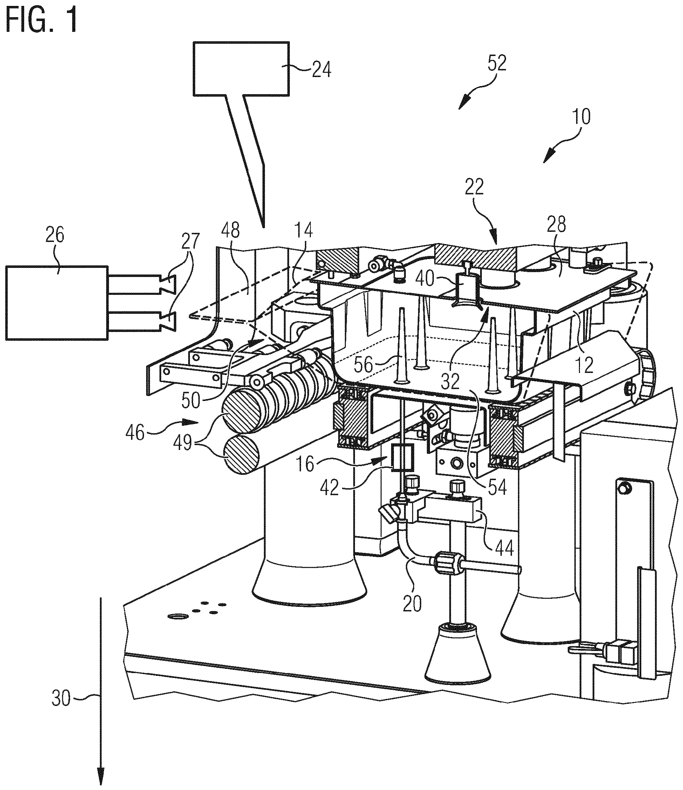

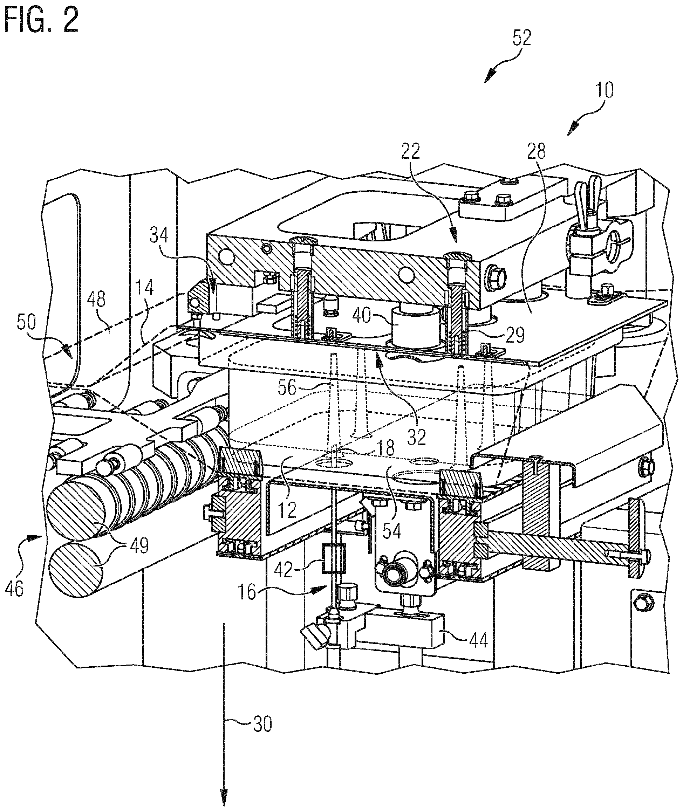

Figur 1 zeigt eine perspektivische Ansicht einer Vorrichtung 10 und Figur 2 zeigt einen Ausschnitt einer perspektivischen Ansicht der Vorrichtung 10 aus Figur 1.

Die Vorrichtung 10 ist zum Entnehmen eines sterilen Gegenstandes 12 aus einem Folienbeutel 14 eingerichtet.

Vorliegend handelt es sich bei dem Gegenstand 12 um einen Tub, also einen wannenförmig ausgebildeten Gegenstand 12. Der Gegenstand 12 weist an seiner senkrecht zur Schwerkraftrichtung 30 orientierten Wandung 54 vier Einbuchtungen 56 auf. Die Einbuchtungen 56 erstrecken sich von der Wandung 54 in das Innere des Gegenstands 12. Die Einbuchtungen 56 erstrecken sich entlang der Schwerkraftrichtung 30, in den Figuren 1 und 2 nach oben.

Die Vorrichtung 10 weist eine Nadeleinheit 16 mit einer hohlen Nadel 18 auf. Die Nadeleinheit 16 bzw. die Nadel 18 sind zum Einstechen in den Folienbeutel 14 eingerichtet.

Die Vorrichtung 10 weist hierzu eine Bewegungseinrichtung 44 auf. Mittels der Bewegungseinrichtung 44 kann die Nadeleinheit 16 mit der Nadel 18 entlang der Schwerkraftrichtung 30 hin und her bewegt werden. So kann die Nadeleinheit 16 mit der Nadel 18 zum Einstechen in den Folienbeutel 14 entgegen der Schwerkraftrichtung 30, in den Figuren 1 und 2 nach oben, bewegt werden.

Dabei taucht die Nadel 18 in eine der vier Einbuchtungen 56 des Gegenstands 12 ein. Mit anderen Worten, die Nadel 18 wird in eine der vier Einbuchtungen 54 des Gegenstands 12 eingeführt.

Zum Herausziehen der Nadel 18 aus dem Folienbeutel 14 kann die Nadeleinheit 16 mit der Nadel 18 in Schwerkraftrichtung 30, in den Figuren 1 und 2 nach unten, bewegt werden.

Die Vorrichtung 10 weist einen an der Nadeleinheit 16 angeordneten Gasanschluss 20 auf. Mittels des Gasanschlusses 20 kann Gas durch die Nadel 18, wenn diese in den Folienbeutel 14 eingestochen hat, in den Folienbeutel 14 eingeleitet werden.

An der Nadeleinheit 16 ist vorliegend eine Filtereinrichtung 42 angeordnet. Mittels der Filtereinrichtung 42 kann das in den Folienbeutel 14 eingeleitete Gas von möglichen Verunreinigungen befreit werden.

Die Vorrichtung 10 weist eine Halteeinrichtung 22 zum Halten des Folienbeutels 14 auf. Die Halteeinrichtung 22 weist eine flächig und eben ausgebildete und beweglich gelagerte Halteeinheit 28 ab. Die Halteeinheit 28 ist senkrecht zur Schwerkraftrichtung 30 orientiert.

Die Halteeinheit 28 weist eine viereckige Form auf und ist vorliegend in Form eines Bleches ausgebildet. Die Halteeinheit 28 ist entlang der Schwerkraftrichtung 30 beweglich ausgebildet. Die Halteeinheit 28 ist in Schwerkraftrichtung 30 mittels vier Schraubenfedern 29 vorgespannt.

Während des Aufblasens des Folienbeutels 14 kontaktiert die Halteeinheit 28 den in Bezug auf die Schwerkraftrichtung 30 oberen Bereich 32 des Folienbeutels 14. Hierdurch wird der obere Bereich 32 des Folienbeutels 14 in eine flache Form gezwungen.

Die Halteeinrichtung 22 weist vorliegend mehrere Saugeinheiten 40 auf, die zum Ansaugen und Halten des Folienbeutels 14 an dessen oberen Bereich 32 ausgebildet sind.

Die Vorrichtung 10 weist eine Schneideinrichtung 24 zum Aufschneiden des Folienbeutels 14 auf.

Die Vorrichtung 10 umfasst eine Entnahmeeinrichtung 26 zum Entnehmen des Gegenstands 12 aus dem aufgeschnittenen Folienbeutel 14.

Die Schneideinrichtung 24 und die Entnahmeeinrichtung 26 sind in Figur 1 lediglich schematisch angedeutet.

Die Vorrichtung 10 weist eine Fixiereinrichtung 46 auf. Die Fixiereinrichtung 46 ist ausgebildet, um einen in Bezug auf die Schwerkraftrichtung 30 seitlichen Bereich 50 des Folienbeutels 14 zu fixieren. Hierzu weist die Fixiereinrichtung 46 zwei Klemmelemente 49 auf. Die beiden Klemmelementen 49 sind ausgebildet, um eine Lasche 48 des Folienbeutels 14, die im seitlichen Bereich 50 des Folienbeutels 14 angeordnet ist, klemmend zu fixieren.

Vorliegend stellen die Vorrichtung 10 und der Gegenstand 12 ein Set 52 dar.

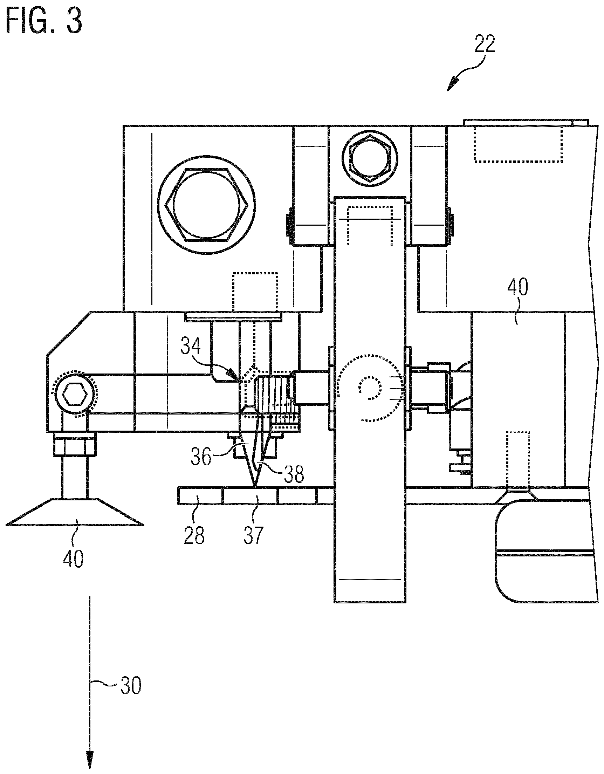

Figur 3 zeigt einen Ausschnitt einer Seitenansicht der Vorrichtung 10 aus Figur 1.

Die Halteeinrichtung 22 weist vorliegend eine Einsticheinrichtung 34 mit einer Einstichnadel 36 zum Einstechen des Folienbeutels 14 auf.

Die Halteeinheit 28 weist eine Öffnung 37 zum Durchleiten der Einstichnadel 36 durch die Halteeinheit 28. Wird also die Halteeinheit 28 nach oben (entgegen der Schwerkraftrichtung 30) bewegt, so durchdringt die Einstichnadel 36 die Halteeinheit 28 durch die Öffnung 37.

Die Einstichnadel 36 weist an ihrer Außenseite eine Außennut 38 auf. Die Außennut 38 ist länglich ausgebildet und erstreckt sich entlang der Längsrichtung der Einstichnadel 36. Sowohl die Einstichnadel 36, als auch die Außennut 38 erstrecken sich somit entlang der Schwerkraftrichtung 30.

Das Entnehmen des Gegenstandes 12 mittels der Vorrichtung 10 aus dem Folienbeutel 14 funktioniert wie folgt:
Der in dem Folienbeutel 14 verpackte Gegenstand 12 wird in der Vorrichtung 10 positioniert. Die Nadeleinheit 16 samt der Nadel 18 werden mittels der Bewegungseinheit 44 in den Figuren 1 und 2 von unten nach oben (also entgegen der Schwerkraftrichtung 30) bewegt. Die Nadel 18 wird somit in die Einbuchtung 56 des Gegenstands 12 eingeführt. Dabei wird der Folienbeutel 14, der die Einbuchtungen 56 überspannt, eingestochen.

Während und/oder nach dem Einstechen wird über den Gasanschluss 20 Gas über die Nadel 18 in der Folienbeutel 14 eingeleitet. Das Gas wird mittels der Filtereinrichtung 42 gefiltert, bevor es in den Folienbeutel 14 über die Nadel 18 eingeleitet wird.

Der Folienbeutel 14 wird durch das eingeleitete Gas aufgeblasen. Dabei trifft der obere Bereich 32 des Folienbeutels 14 auf die Halteeinheit 28 und drückt diese entgegen der Vorspannung der vier Spiralfedern 29 nach oben (also entgegen der Schwerkraftrichtung 30).

Hierdurch wird der obere Bereich 32 des Folienbeutels 14 in eine flache, der Halteeinheit 28 entsprechende, Form gezwungen. Aufgrund der nun flachen Form des oberen Bereichs 32 können die Saugeinheiten 40 der Halteeinrichtung 22 besser an dem oberen Bereich 32 des Folienbeutels 14 angreifen und diesen besser ansaugen bzw. halten.

Durch weiteres Einleiten des Gases durch die Nadel 18 wird der Folienbeutel 14 weiter aufgeblasen. Der obere Bereich 32 des Folienbeutels 14 drückt die Halteeinheit 28 weiter nach oben (entgegen der Schwerkraftrichtung 30).

Wird der Folienbeutel 14 über ein gewünschtes Maß hinaus aufgeblasen, so schiebt der Folienbeutel 14 mit seinem oberen Bereich 32 die Halteeinheit 28 über die Einstichnadel 36. Dabei wird die Halteeinheit 28 mit der Öffnung 37 über die Einstichnadel 36 bewegt, so dass die Einstichnadel 36 aus der Öffnung 37 nach unten (in Schwerkraftrichtung 30) herausragt. Hierbei wird der Folienbeutel 14 in seinem oberen Bereich 32 mittels der aus der Öffnung 37 herausragenden Einstichnadel 36 eingestochen.

Das Gas kann nun aus dem Folienbeutel 14 durch die Einstichstelle der Einstichnadel 36 entweichen. Das Entweichen des Gases wird durch die Außennut 38 begünstigt. Damit kann gewährleistet werden, dass der Folienbeutel 14 nicht über ein gewünschtes Maß hinaus aufgeblasen wird und damit stets eine gewünschte Form, insbesondere in seinem oberen Bereich 32, beibehält.

Der Folienbeutel 14 wird mittels der Saugeinheiten 40 der Halteeinrichtung 22 in seinem oberen Bereich 32 gehalten.

Da die gewünschte Form des Folienbeutels 14 beibehalten wird, wird das Aufschneiden des Folienbeutels 14 mittels der Schneideinrichtung 24, als auch das Fixieren des Folienbeutels 14 mittels der Fixiereinrichtung 46, sowie das Halten des Folienbeutels 14 mittels der Halteeinrichtung 22 erleichtert.

Nach dem Aufblasen des Folienbeutels 14 auf die gewünschte Form, wird der Folienbeutel 14 mittels der Fixiereinrichtung 46 fixiert. Hierzu wird die Lasche 48 des Folienbeutels 14 mittels der beiden Klemmelemente 49 klemmend fixiert. Hierdurch wird der Bereich zwischen der Lasche 48 und dem oberen Bereich 32 des Folienbeutels 14 gestreckt, sodass der Folienbeutel 14 in diesem gestreckten Bereich besonders gut mittels der Schneideinrichtung 24 aufgeschnitten werden kann.

Nach dem Aufschneiden des Folienbeutels 14 wird der Gegenstand 12 mittels der Entnahmeeinrichtung 26 aus dem Folienbeutel 14 entnommen. Hierzu weist die Entnahmeeinrichtung 26 mehrere Saugelemente 27 auf. Die Entnahmeeinrichtung 26 bewegt die Saugelemente 27 in den aufgeschnittenen Folienbeutel 14 hinein. Die Saugelemente 27 greifen an einer Wandung des Gegenstands 12 an und saugen sich daran fest. Hierdurch wird der Gegenstand 12 an der Entnahmeeinrichtung 26 bzw. deren Saugelementen 27 fixiert. Anschließend werden die Saugelemente 27 und damit der Gegenstand 12 aus dem aufgeschnittenen Folienbeutel 14 bewegt.

Vor der Entnahme des Gegenstands 12 aus dem Folienbeutel 14 und nach dem Aufschneiden des Folienbeutels 14 wird die Nadel 18 aus der Einbuchtung 56 des Gegenstands 12 entfernt, in dem die Bewegungseinrichtung 44 die Nadeleinheit 16 samt der Nadel 18 nach unten (in Schwerkraftrichtung 30) bewegt. Andernfalls würde die in der Einbuchtung 56 angeordnete Nadel 18 eine Entnahme des Gegenstands 12 behindern.

Der aufgeschnittene und leere Folienbeutel 14 kann nach der Entnahme des Gegenstands 12 entsorgt werden. Dies kann bspw. mittels der Fixiereinrichtung 46 umgesetzt werden, die den fixierten, aufgeschnittenen und leeren Folienbeutels 14 an einen Entsorgungsort transportiert. Hierzu kann die Fixiereinrichtung 46 entsprechend bewegbar ausgebildet sein.

## Patentansprüche

1. Vorrichtung (10) zum Entnehmen eines sterilen Gegenstandes (12), insbesondere eines Tubs, aus einem Folienbeutel (14), umfassend:
- eine Nadeleinheit (16) mit einer hohlen Nadel (18) zum Einstechen in den Folienbeutel (14),
- einen an der Nadeleinheit (16) angeordneten Gasanschluss (20) zum Einleiten von Gas über die hohle Nadel (18) in den Folienbeutel (14),
- eine Halteeinrichtung (22) zum Halten des Folienbeutels (14),
- eine Schneideinrichtung (24) zum Aufschneiden des Folienbeutels (14), und
- eine Entnahmeeinrichtung (26) zum Entnehmen des Gegenstands (12) aus dem aufgeschnittenen Folienbeutel (14),
**dadurch gekennzeichnet, dass**
die Halteeinrichtung (22) mindestens eine an der Halteeinrichtung (22) flächig, insbesondere eben, ausgebildete und beweglich gelagerte Halteeinheit (28) zur Kontaktierung des Folienbeutels (14) aufweist.

2. Vorrichtung (10) nach Anspruch 1, **dadurch gekennzeichnet, dass** die Halteeinheit (28) als Blech ausgebildet ist.

3. Vorrichtung (10) nach Anspruch 1 oder 2, **dadurch gekennzeichnet, dass** die Halteeinheit (28) senkrecht zur Schwerkraftrichtung (30) orientiert ist und insbesondere zur Kontaktierung eines in Bezug auf die Schwerkraftrichtung (30) oberen Bereichs (32) des Folienbeutels (14) ausgebildet ist.

4. Vorrichtung (10) nach einem der voranstehenden Ansprüche, **dadurch gekennzeichnet, dass** die Halteeinrichtung (22) mindestens eine Einsticheinrichtung (34) mit einer Einstichnadel (36) zum Einstechen des Folienbeutels (14), insbesondere im in Bezug auf die Schwerkraftrichtung (30) oberen Bereich (32) des Folienbeutels (14), und zum Ausleiten des durch die Nadel (18) in den Folienbeutel (14) eingeleiteten Gases aus dem Folienbeutel (14) aufweist.

5. Vorrichtung (10) nach dem voranstehenden Anspruch, **dadurch gekennzeichnet, dass** die Einstichnadel (36) mindestens eine Außennut (38) zum Ausleiten des durch die Nadel (18) in den Folienbeutel (14) eingeleiteten Gases aus dem Folienbeutel (14) aufweist.

6. Vorrichtung (10) nach einem der voranstehenden Ansprüche, **dadurch gekennzeichnet, dass** die Halteeinrichtung (22) mindestens eine Saugeinheit (40) zum Ansaugen und/oder Halten des Folienbeutels (14) umfasst, insbesondere wobei die Saugeinheit (40) zur Kontaktierung des in Bezug auf die Schwerkraftrichtung (30) oberen Bereichs (32) des Folienbeutels (14), und vorzugsweise als Saugnapf, ausgebildet ist.

7. Vorrichtung (10) nach einem der voranstehenden Ansprüche, **dadurch gekennzeichnet, dass** die Nadeleinheit (16) eine Filtereinrichtung (42) zum Filtern des in den Folienbeutel (14) mittels der Nadel (18) eingeleiteten Gases umfasst, wobei die Filtereinrichtung (42) insbesondere innerhalb der Nadel (18) und/oder innerhalb des Gasanschlusses (20) angeordnet ist.

8. Vorrichtung (10) nach einem der voranstehenden Ansprüche, **dadurch gekennzeichnet, dass** die Vorrichtung (10) eine Bewegungseinrichtung (44) umfasst, wobei die Bewegungseinrichtung (44) eingerichtet ist, um die Nadeleinheit (16) und/oder die Nadel (18) zum Einstechen der Nadel (18) in den Folienbeutel (14) und zum Zurückziehen der Nadel (18) aus dem Folienbeutel (14) zu bewegen.

9. Vorrichtung (10) nach einem der voranstehenden Ansprüche, **dadurch gekennzeichnet, dass** die Vorrichtung (10) eine Fixiereinrichtung (46) zur Fixierung eines, insbesondere eine Lasche (48) des Folienbeutels (14) aufweisenden, in Bezug auf die Schwerkraftrichtung (30) seitlichen Bereichs (50) des Folienbeutels (14) aufweist.

10. Vorrichtung (10) nach dem voranstehenden Anspruch, **dadurch gekennzeichnet, dass** die Fixiereinrichtung (46) mindestens zwei Klemmelemente (49) umfasst, die ausgebildet sind, um den Folienbeutel (14), insbesondere den in Bezug auf die Schwerkraftrichtung (30) seitlichen Bereich (50) des Folienbeutels (14), vorzugsweise an der Lasche (48) des Folienbeutels (14), klemmend zu fixieren.

11. Set (52) umfassend eine Vorrichtung (10) nach einem der voranstehenden Ansprüche und mindestens einen Gegenstand (12), insbesondere einen Tub, der von einem Folienbeutel (14) umgeben ist, wobei der Gegenstand (12), insbesondere an einer den Folienbeutel (14) kontaktierenden Wandung (54) des Gegenstands (12), mindestens eine Einbuchtung (56) aufweist, insbesondere wobei die Einbuchtung (56) von dem Folienbeutel (14) überspannt wird, wobei die Vorrichtung (10) und/oder der Gegenstand (12) derart ausgebildet sind, dass die Nadel (18) zum Einstechen in den Folienbeutel (14) zumindest teilweise in die Einbuchtung (56) des Gegenstands (12) eingeführt wird.

12. Set (52) nach Anspruch 11, **dadurch gekennzeichnet, dass** die Halteeinheit (28), insbesondere in Blickrichtung entlang der Schwerkraftrichtung (30), den Gegenstand (12) an mindestens einer Seite des Gegenstands (12), insbesondere an allen Seiten des Gegenstands (12), überragt.

13. Verfahren zum Entnehmen eines sterilen Gegenstandes (12), insbesondere eines Tubs, aus einem Folienbeutel (14), umfassend die Schritte:
- Bereitstellen des in dem Folienbeutel (14) angeordneten Gegenstandes (12);
- Einstechen des Folienbeutels (14) mittels einer hohlen Nadel (18);
- Einleiten von Gas über die Nadel (18) in den Folienbeutel (14);
- Halten des Folienbeutels (14) mittels einer Halteeinrichtung (22), wobei die Halteeinrichtung (22) mindestens eine an der Halteeinrichtung (22) flächig, insbesondere eben, ausgebildete und beweglich gelagerte Halteeinheit (28) zur Kontaktierung des Folienbeutels (14) aufweist;
- Kontaktieren, insbesondere eines in Bezug auf die Schwerkraftrichtung (30) oberen Bereichs (32), des Folienbeutels (14) mittels der Halteeinheit (28);
- Nach dem Kontaktieren des Folienbeutels (14) mittels der Halteeinheit (28), weiteres Einleiten von Gas über die Nadel (18) in den Folienbeutel (14), so dass dieser weiter aufgeblasen wird und, insbesondere mit seinem in Bezug auf die Schwerkraftrichtung (30) oberen Bereich (32), die Halteeinheit (28), insbesondere entgegen der Schwerkraftrichtung (30), bewegt.

14. Verfahren nach Anspruch 13, **dadurch gekennzeichnet, dass** das Verfahren den Schritt umfasst:
- Während des Einleiten von Gas über die Nadel (18) in den Folienbeutel (14), Einstechen des Folienbeutels (14) zum Ausleiten des durch die Nadel (18) in den Folienbeutel (14) eingeleiteten Gases aus dem Folienbeutel (14), insbesondere mittels einer Einstichnadel (36), insbesondere in dem in Bezug auf die Schwerkraftrichtung (30) oberen Bereich (32) des Folienbeutels (14).

15. Verfahren nach Anspruch 13 oder 14, **dadurch gekennzeichnet, dass** das Verfahren die Schritte umfasst:
- Aufschneiden des durch die Nadel (18) aufgeblasenen und mittels der Halteeinrichtung (22) gehaltenen Folienbeutels (14), insbesondere mittels einer Schneideinrichtung (24);
- Entnehmen des Gegenstands (12) aus dem Folienbeutel (14), insbesondere mittels einer Entnahmeeinrichtung (26),
- insbesondere Entsorgen des leeren und
aufgeschnittenen Folienbeutels (14).

16. Verfahren nach einem der Ansprüche 13 bis 15, **dadurch gekennzeichnet, dass** zur Durchführung des Verfahrens eine Vorrichtung (10) nach einem der Ansprüche 1 bis 10 oder ein Set (52) nach Anspruch 11 oder 12 verwendet wird.
